(19) Europäisches Patentamt
European Patent Office
Office européen des brevets

(11) Numéro de publication : **0 565 417 A1**

(12) # DEMANDE DE BREVET EUROPEEN

(21) Numéro de dépôt : **93400872.3**

(22) Date de dépôt : **05.04.93**

(51) Int. Cl.$^5$ : **A61K 7/00,** A61K 7/13, C09C 3/08, C09B 67/08, C09B 63/00

(30) Priorité : **08.04.92 FR 9204313**

(43) Date de publication de la demande : **13.10.93 Bulletin 93/41**

(84) Etats contractants désignés : **AT BE CH DE DK ES FR GB GR IE IT LI NL PT SE**

(71) Demandeur : **L'OREAL**
**14, Rue Royale**
**F-75008 Paris (FR)**

(72) Inventeur : **Philippe, Michel**
**3, rue de l'Aubépine**
**F-92160 Antony (FR)**
Inventeur : **Hocquaux, Michel**
**70, rue du Rendez-vous**
**F-75012 Paris (FR)**
Inventeur : **Bordier, Thierry**
**7, résidence Jean Monnet**
**F-93190 Livry-Gargan (FR)**

(74) Mandataire : **Casalonga, Axel et al**
**BUREAU D.A. CASALONGA - JOSSE**
**Morassistrasse 8**
**D-80469 München (DE)**

(54) **Pigments comportant un support inorganique et le produit de réaction d'un dérivé indolique et d'un dérivé quinonique, leur procédé de préparation et leur utilisation en cosmétique.**

(57) La présente invention concerne des pigments se présentant sous forme de particules à base d'un support inorganique et de produits de réaction d'un dérivé indolique et d'un dérivé quinonique.

Elle concerne également un procédé pour leur préparation, leur utilisation dans l'industrie alimentaire, les peintures et en cosmétique, les compositions cosmétiques en comportant et leur utilisation pour le maquillage, la protection contre les UV et pour la coloration des cheveux ou des ongles.

EP 0 565 417 A1

La présente invention a pour objet de nouveaux pigments se présentant sous forme de particules à base d'un support inorganique et de produits de réaction d'un dérivé indolique et d'un dérivé quinonique, leur procédé de préparation, leur utilisation dans l'industrie alimentaire, les peintures et plus particulièrement en cosmétique, ainsi que les compositions cosmétiques les contenant.

On a déjà décrit dans l'état de la technique différentes réactions mettant en présence un dérivé indolique et un dérivé quinonique, telles qu'une benzoquinone, une naphtoquinone. De telles réactions sont décrites plus particulièrement dans la littérature par MOHLAU & REDLICH (Ber., 44, 3605, 1911), (K.K. PRASAD, Tetrahedron Letters, 1361, 1974, G. PROTA, Gazetta Chim. Ital. 119, 153, 1989).

La demanderesse a également décrit l'utilisation de colorants résultant du couplage d'un dérivé indolique avec une quinone pour la teinture des fibres kératiniques FR-A-2.626.173, EP-A-376776.

La demanderesse a découvert que la réaction de couplage d'un dérivé indolique et d'un dérivé quinonique tel que défini ci-après, en présence d'une charge minérale, permettait d'obtenir des particules colorées, sous forme de pigments, dont la couleur est celle du colorant obtenu par couplage du dérivé indolique et du dérivé quinonique, ce qui n'est pas toujours le cas si l'on réalise la simple adsorption du colorant sur la charge minérale.

La demanderesse a par ailleurs constaté que les particules ainsi obtenues pouvaient être utilisées comme pigments et pouvaient présenter une granulométrie beaucoup plus homogène et plus faible que les particules pouvant être obtenues par des procédés mettant en oeuvre une adsorption du colorant résultant du couplage du dérivé indolique avec le dérivé quinonique sur la particule minérale.

On a constaté enfin que la formation in situ de telles particules colorées permettait de choisir la granulométrie du produit final en fonction de la granulométrie de la charge minérale de départ et ceci selon l'utilisation envisagée.

Elle a enfin découvert que l'utilisation de telles particules à titre de pigments dans les compositions cosmétiques, notamment dans les compositions de maquillage, telles que les poudres ou les fonds de teint ou encore les rouges à lèvres, les mascaras, les fards à paupières, les vernis à ongles, est particulièrement intéressante compte tenu des propriétés apportées par les particules préparées conformément à l'invention, tant en ce qui concerne leurs propriétés colorantes qu'en ce qui concerne leur homogénéité.

Un objet de l'invention est donc constitué par -des particules inorganiques comportant dans et/ou sur ces particules, le produit de réaction d'un dérivé indolique et d'un dérivé quinonique.

Un autre objet de l'invention est constitué par le procédé de préparation de ces particules colorées.

Un autre objet de l'invention est constitué par l'utilisation de ces particules comme pigments, dans l'industrie alimentaire, des peintures et en particulier dans le domaine cosmétique.

L'invention a également pour objet des compositions cosmétiques, notamment de maquillage mettant en oeuvre de tels pigments.

D'autres objets de l'invention apparaîtront à la lecture de la description et des exemples qui suivent.

Les particules inorganiques colorées conformes à l'invention sont essentiellement caractérisées par le fait qu'elles résultent de la réaction en présence des particules inorganiques d'un dérivé indolique et d'un dérivé quinonique dans un milieu approprié pour cette réaction.

Les dérivés indoliques utilisés conformément à l'invention répondent à la formule (I) :

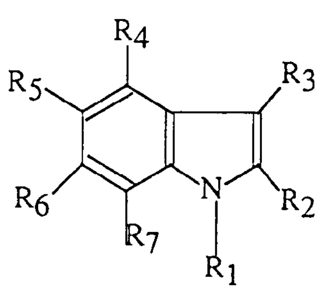

(I)

dans laquelle :

$R_1$ représente un atome d'hydogène ou un groupement alkyle inférieur en $C_1$-$C_4$;

$R_2$ et $R_3$, identiques ou différents, représentent un atome d'hydrogène, un groupement alkyle en $C_1$-$C_4$, phényle ou carboxyle;

$R_4$, $R_5$, $R_5$ et $R_7$, identiques ou différents, représentent un atome d'hydrogène, d'halogène, hydroxyle, un groupement alkyle ou alcoxy en $C_1$-$C_4$, alcoxy($C_1$-$C_4$)carbonyle, acyle en $C_2$-$C_4$, acyl($C_2$-$C_4$)oxy, cyano, amino éventuellement substitué par alkyle en $C_1$-$C_4$ ou hydroxyalkyle en $C_1$-$C_4$, $NO_2$, benzyloxy, $R_5$ et $R_6$ pouvant former un cycle méthylènedioxy, un seul au plus des radicaux $R_4$ à $R_7$ peut désigner halogène, $NO_2$, alcoxy-

carbonylé, acyle, cyano, amino, deux au plus des radicaux $R_4$ à $R_7$ peuvent désigner OH, alcoxy, benzyloxy ou acyloxy.

Les dérivés quinoniques sont choisis parmi les 1,4 et 1,2-benzoquinones ou naphtoquinones.

Les 1,2-benzoquinones sont choisis parmi les composés répondant à la formule (II) :

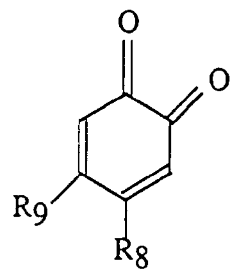

(II)

dans laquelle $R_8$ et $R_9$ désignent, indépendamment l'un de l'autre, un atome d'hydrogène ou d'halogène, un radical alkyle en $C_1$-$C_4$ ou alcoxy en $C_1$-$C_4$ ou le groupe $SO_3M$, M représentant un atome d'hydrogène, un métal alcalin, ammonium ou un reste d'amine.

Les 1,4-benzoquinones répondent à la formule (III) :

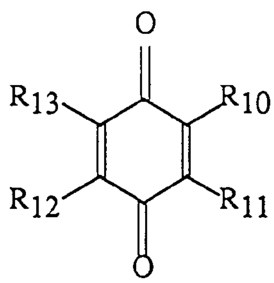

(III)

dans laquelle $R_{10}$ désigne un atome d'hydrogène, un atome d'halogène, un radical alkyle en $C_1$-$C_4$, alcoxy en $C_1$-$C_4$, hydroxyalkyle en $C_1$-$C_4$, acyl($C_1$-$C_4$)amino, alcoxy($C_1$-$C_4$)carbonyle, hydroxyalcoxy($C_1$-$C_4$), carboxyalkyle($C_1$-$C_4$), carboxyalcoxy($C_1$-$C_4$), alcoxy($C_1$-$C_4$) alkyle($C_1$-$C_4$), hydroxyalkyl($C_1$-$C_4$)thio, polyhydroxyalkyl($C_1$-$C_4$) thio, carboxyalkyl($C_1$-$C_4$)thio, alkyl($C_1$-$C_4$)thio, mono- ou dialkyl ($C_1$-$C_4$)amino, hydroxyalkyl($C_1$-$C_4$)thioalkyl($C_1$-$C_4$), alkyl($C_1$-$C_4$) thioalkyle($C_1$-$C_4$), $SO_3M$, M ayant les significations indiquées ci-dessus;

$R_{11}$, $R_{12}$, $R_{13}$ désignent, indépendamment l'un de l'autre, un atome d'hydrogène, d'halogène, un radical alkyle en $C_1$-$C_4$, alcoxy en $C_1$-$C_4$, hydroxyalkyl($C_1$-$C_4$)thio, acylamino,

et au moins l'un des radicaux $R_{10}$ à $R_{13}$ désigne hydrogène.

Les 1,2 et 1,4-naphtoquinones sont choisi parmi les composés répondant aux formules (IV) et (V) :

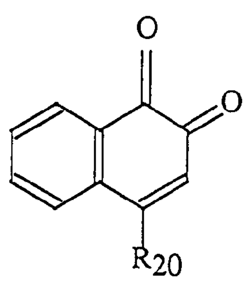

(IV)

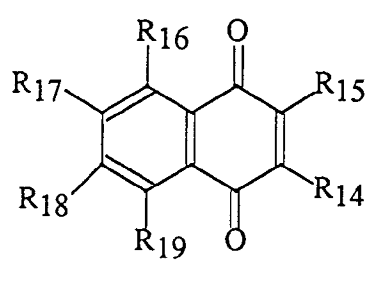

(V)

dans laquelle :

$R_{16}$ à $R_{19}$, identiques ou différents, désignent un atome d'hydrogène, un groupement OH, un groupement alcoxy en $C_1$-$C_4$, deux groupements au plus pouvant désigner OH;

$R_{14}$ et $R_{15}$ désignent un atome d'hydrogène, un groupement OH, un atome d'halogène, un radical alkyle en $C_1$-$C_{12}$, alcoxy en $C_1$-$C_4$, phényle, thiophényle, adamantylméthyle, adamantylamino, alkyl($C_1$-$C_4$)amino, pipéridyle, morpholinyle;

$R_{20}$ représente un atome d'hydrogène ou un groupe $SO_3M$, M ayant les significations indiquées pour la formule (II).

Les dérivés indoliques plus particulièrement préférés sont choisis parmi l'indole, le N-méthylindole, le 2-méthylindole, le 3-méthylindole, le 2-phénylindole, le 2-carboxyindole.

Les dérivés quinoniques plus particulièrement préférés sont choisis parmi la 1,4-benzoquinone, la 1,4-naphtoquinone, la 5-hydroxy 1,4-naphtoquinone, la 2-hydroxy 1,4-naphtoquinone, la 5-hydroxy 2-méthyl 1,4-naphtoquinone, la 3-méthoxy 2-bromo 1,4-naphtoquinone, la 2,5-dihydroxy 1,4-naphtoquinone, le 4-sulfonate 1,2-naphtoquinone de sodium.

Les particules inorganiques utilisables conformément à l'invention, sont choisies parmi des particules solides, à base d'oxydes de magnésium, de fer, de silicium, d'aluminium, de titane, de chrome, de zinc, de manganèse, le carbonate de magnésium, l'oxychlorure de bismuth.

A titre représentatif, on peut citer le talc, les alumines, le kaolin, le carbonate de magnésium, les oxydes de titane, les oxydes de zinc, les oxydes de chrome, la silice, les oxydes de fer, le mica, l'oxychlorure de bismuth.

Les particules inorganiques préférentiellement utilisées, conformes à l'invention, sont choisies parmi les particules de talc, d'alumine, de silice.

Les particules inorganiques utilisées conformément à l'invention ont une granulométrie généralement comprise entre 0,1 et 200 $\mu$m et de préférence entre 0,5 et 100 $\mu$m

La demanderesse a découvert que des particules inorganiques colorées in situ par réaction de dérivés indoliques et de dérivés quinoniques en présence de particules inorganiques, leur conférait des propriétés particulièrement intéressantes par rapport à des particule colorées obtenues par adsorption sur le même type de particules du ou des produits colorés provenant de la réaction préalable du même dérivé indolique et du même dérivé quinonique.

C'est ainsi qu'en faisant réagir la 1,4-naphtoquinone avec le 2-méthylindole en présence de talc, on isole un produit sous forme de particules ayant une coloration violette, alors qu'en faisant adsorber le colorant résultant de la réaction de la 1,4-naphtoquinone avec le 2-méthylindole sur les mêmes particules de talc, on aboutit à un produit sous forme de particules ayant une coloration grise.

De même, en faisant réagir la 1,4-naphtoquinone avec l'indole en présence d'alumine neutre, on isole une poudre ayant une coloration rouge et une répartition des particules très homogène, alors qu'en faisant adsorber le colorant résultant de la réaction de la 1,4-naphtoquinone avec l'indole sur l'alumine neutre, on aboutit à une poudre ayant une couleur rouge, mais très peu homogène au niveau de la répartition de la granulométrie des particules.

La demanderesse a constaté qu'il était possible grâce au procédé conforme à l'invention, de préparer des poudres comportant des particules ayant une répartition homogène et ainsi choisir la granulométrie de ces particules suivant l'utilisation envisagée. Il a été ainsi possible de préparer des particules utilisables comme pigments, de même coloration, mais de granulométrie différente en utilisant des charges de départ différentes, adaptées à l'usage que l'on souhaite en faire.

Les dérivés indoliques et quinoniques utilisés dans le procédé conforme à l'invention, peuvent être utilisés dans des proportions variables comprises entre 1 et 1000% par rapport aux particules inorganiques mises en oeuvre et de préférence entre 10 et 500% en poids.

La réaction des dérivés indoliques avec le dérivé quinonique s'effectue de préférence dans un milieu solvant, en particulier dans un milieu éthanolique ou dans un mélange eau-acide acétique ou encore dans l'acide acétique pur.

Le rapport molaire entre le dérivé quinonique et le dérivé indolique peut varier dans de larges limites, par exemple entre 0,05 et 10 et de préférence entre 0,1 et 5.

Les particules inorganiques colorées ou pigments ainsi préparés, peuvent être additionnés dans les supports cosmétiques classiques dans des proportions suffisantes pour leur conférer la coloration souhaitée et généralement à une concentration comprise entre 0,1 et 50% et de préférence entre 2 et 40% par rapport au poids total de la composition pour conduire à des compositions de coloration temporaire pour cheveux ou des produits de maquillage, notamment des cils, des sourcils ou de la peau, tels que des fards à paupières, fards à joues, ligneurs encore appelés "eye-liners", mascaras pour les cils et les sourcils, rouge à lèvres, ou des produits dits "vernis à ongles". Ces supports cosmétiques sont connus en eux-mêmes.

Ces compositions peuvent se présenter sous forme de lotion, de lotion épaissie, de gel, de crème, de pommade, de poudre, de stick, de dispersion, de dispersion liposomale, éventuellement être conditionnées en aérosol.

Les compositions utilisées pour le maquillage de la peau, des cils et des sourcils peuvent notamment se présenter sous forme solide ou pâteuse, anhydre ou aqueuse. Il peut s'agir dans ce dernier cas d'émulsions huile-dans-eau ou eau-dans-huile ou encore de suspensions.

Lorsque ces compositions sont utilisées sous forme d'émulsions, elles contiennent en outre des agents tensio-actifs bien connus dans l'état de la technique, notamment des agents tensio-actifs anioniques, non-ioniques, cationiques, amphotères ou leurs mélanges.

Les compositions de maquillage peuvent également contenir des corps gras, des solvants organiques, des silicones, des épaississants, des adoucissants, des filtres solaires, des parfums, des conservateurs, des antioxydants, des charges, des séquestrants, des polymères anioniques, cationiques, non-ioniques ou amphotères ainsi que leurs mélanges, des agents alcalinisants ou acidifiants.

Les corps gras peuvent être constitués par une huile ou une cire ou leurs mélanges. Les huiles sont choisies de préférence parmi les huiles animales, végétales, minérales ou de synthèse et notamment l'huile de palme hydrogénée, l'huile de ricin hydrogénée, l'huile de vaseline, l'huile de paraffine, l'huile de Purcellin et la lanoline.

Les cires sont choisies parmi les cires animales, fossiles, végétales, minérales ou synthétiques, parmi lesquelles on peut citer les cires d'abeilles, les cires de Carnauba, de Candellila, de canne à sucre, du Japon, les ozokérites, la cire de Montan, les cires microcristallines, les paraffines.

Les compositions conformes à l'invention se caractérisent par une stabilité remarquable et par une bonne innocuité.

Les maquillages obtenus résistent bien aux agents atmosphériques ainsi qu'à l'eau.

Les compositions conformes à l'invention peuvent également contenir en plus des pigments tels que définis ci-dessus, d'autres pigments généralement utilisés en cosmétique et notamment des pigments nacrés et/ou nacrants permettant de varier les colorations susceptibles d'être obtenues ou d'augmenter leur protection vis-à-vis du rayonnement ultraviolet. On peut utiliser notamment des pigments métalliques tels que les oxydes de titane, de zinc, de cérium ou de zirconium.

Une forme de réalisation particulière consiste à utiliser en plus des nanopigments ayant une granulométrie inférieure à 100 nanomètres.

L'invention a également pour objet un procédé de maquillage de la peau, de protection de l'épiderme humain contre les effets néfastes des rayonnements UV, de coloration des cheveux ou des ongles, mettant en oeuvre les compositions et comportant au moins en partie à titre de pigments, les produits sous forme de particules inorganiques colorées tels que définis ci-dessus.

Les exemples suivants sont destinés à illustrer l'invention sans pour autant présenter un caractère limitatif.

## EXEMPLES DE PREPARATION

### EXEMPLE DE PREPARATION 1

Dans un tricol de 50 ml, muni d'un réfrigérant, d'un thermomètre et d'une agitation magnétique, on place 0,9 g (5,69 mmoles) de 1,4-naphtoquinone et 0,9 g de talc dans 9 ml d'acide acétique. On chauffe sous agitation à reflux puis on ajoute 0,66 g d'indole (leq.) Après 24 heures, on refroidit, on filtre, et on lave le pigment dans des solvants de polarités différentes (eau, éthanol, acétone) jusqu'à l'obtention d'un filtrat non coloré.

On obtient un pigment rouge.

### EXEMPLE DE PREPARATION 2

Dans un tricol de 100 ml, muni d'un réfrigérant, d'un thermomètre et d'une agitation magnétique, on place 3,5 g (22,1 mmoles) de 1,4-naphtoquinone et 3,5 g de talc dans 35 ml d'acide acétique. On chauffe sous agitation à la température de 70°C puis on ajoute 0,4 g (0,154 eq.) d'indole. Après 6 heures, on refroidit, on filtre et on lave le pigment dans des solvants de polarités différentes (eau, éthanol, acétone) jusqu'à l'obtention d'un filtrat non coloré.

On obtient un pigment rouge.

### EXEMPLE DE PREPARATION 3

Dans un tricol de 250 ml, muni d'une agitation mécanique, d'un réfrigérant et d'un thermomètre, on place 10 g (63,2 mmoles) de 1,4-naphtoquinone et 50 g d'alumine neutre dans 200 ml d'un mélange acide acétique-eau (1-1). On chauffe sous agitation à la température de 70°C puis on ajoute 3 g d'indole (0,4 eq). Après 24 heures, on refroidit, on filtre et on lave le pigment dans des solvants de polarités différentes (eau, éthanol, acétone) jusqu'à l'obtention d'un filtrat non coloré.

On obtient un pigment rouge.

EXEMPLE DE PREPARATION 4

Dans un tricol de 50 ml, muni d'un réfrigérant, d'un thermomètre et d'une agitation magnétique, on place 0,9 g (5,69 mmoles) de 1,4-naphtoquinone et 1,8 g d'alumine neutre dans 18 ml d'acide acétique. On agite à la température ambiante pendant 10 minutes, puis on ajoute 0,45 g d'indole (0,66 eq). Après 48 heures, on filtre et on lave le pigment dans des solvants de polarités différentes (eau, éthanol, acétone) jusqu'à l'obtention d'un filtrat non coloré.

On obtient un pigment rouge.

EXEMPLE DE PREPARATION 5

Dans un tricol de 50 ml, muni d'un réfrigérant, d'un thermomètre et d'une agitation magnétique, on place 1,6 g de 1,4-naphtoquinone (10 mmoles) et 1,6 g de talc dans 16 ml d'éthanol. On chauffe sous agitation à la température de 70°C puis on ajoute 0,52 g (0,4 eq) de 2-méthylindole. Après 24 heures, on refroidit, on filtre et on lave le pigment dans des solvants de polarités différentes (éthanol, acétone) jusqu'à l'obtention d'un filtrat non coloré.

On obtient un pigment violet.

EXEMPLE DE PREPARATION 6

Dans un tricol de 100 ml, muni d'un réfrigérant, d'un thermomètre et d'une agitation magnétique, on place 1,8 g (11,4 mmoles) de 1,4-naphtoquinone et 3,6 g d'alumine acide dans 72 ml d'éthanol. On chauffe sous agitation à la température de 70°C puis on ajoute 1 g (0,66 eq) de 2-méthylindole. Après 48 heures, on refroidit, on filtre et on lave le pigment dans des solvants de polarités différentes (éthanol, acétone) jusqu'à l'obtention d'un filtrat non coloré.

On obtient un pigment violet.

EXEMPLE DE PREPARATION 7

Dans un tricol de 50 ml, muni d'un réfrigérant, d'un thermomètre et d'une agitation magnétique, on place 0,5 g (2,66 mmoles) de 5-hydroxy 2-méthyl 1,4-naphtoquinone et 1g d'alumine acide dans 20 ml d'éthanol. On chauffe sous agitation à la température de 70°C puis on ajoute 0,35 g (1 eq) de 2-méthylindole. Après 24 heures, on refroidit, on filtre et on lave le pigment dans des solvants de polarités différentes (éthanol, acétone) jusqu'à l'obtention d'un filtrat non coloré.

On obtient un pigment violet.

EXEMPLE DE PREPARATION 8

Selon le même protocole opératoire que l'exemple 6, on obtient, à partir du 5-hydroxy 2-méthyl 1,4-naphtoquinone et de l'indole (1 eq), un pigment violet.

EXEMPLE DE PREPARATION 9

Selon le même protocole opératoire que l'exemple 5, on obtient, à partir du 2-bromo 3-méthoxy 1,4-naphtoquinone et du 2-méthylindole (3 eq), un pigment violet.

EXEMPLE DE PREPARATION 10

Selon le même protocole opératoire que l'exemple 11, on obtient, à partir du 2-hydroxy 1,4-naphtoquinone et du 2-méthylindole (2 eq), un pigment violet.

EXEMPLE DE PREPARATION 11

Dans un tricol de 50 ml, muni d'un réfritérant, d'un thermomètre et d'une agitation magnétique, on place 0,9 g de 5-hydroxy 1,4-naphtoquinone (5,2 mmoles) et 0,9 g de talc dans 9 ml d'acide acétique. On chauffe sous agitation à la température de 70°C puis on ajoute 0,6 g (1 eq) d'indole. Après 24 heures, on refroidit, on

filtre et on lave le pigment dans des solvants de polarités différentes (eau, éthanol, acétone) jusqu'à l'obtention d'un filtrat non coloré.

On obtient un pigment noir.

EXEMPLE DE PREPARATION 12

Dans un tricol de 50 ml, muni d'un réfrigérant, d'un thermomètre et d'une agitation magnétique, on place 0,9 g (5,2 mmoles) de 5-hydroxy 1,4-naphtoquinone et 1,8 g d'alumine acide dans 36 ml d'éthanol. On chauffe sous agitation à la température de 70°C puis on ajoute 0,41 g (0,66 eq) d'indole. Après 24 heures, on refroidit, on filtre et on lave le pigment dans des solvants de polarités différentes (éthanol, acétone) jusqu'à l'obtention d'un filtrat non coloré.

On obtient un pigment noir.

EXEMPLE DE PREPARATION 13

Selon le même protocole opératoire que l'exemple 12, on obtient, à partir du 5-hydroxy 1,4-naphtoquinone et du 2-carboxyindole (0,66 eq), un pigment noir.

EXEMPLE DE PREPARATION 14

Selon le même protocole opératoire que l'exemple 12, on obtient, à partir du 2,5-dihydroxy 1,4-naphtoquinone et du 2-méthylindole (0,66 eq), un pigment noir.

EXEMPLE DE PREPARATION 15

Selon le même protocole opératoire que l'exemple 11, on obtient, à partir du 5-hydroxy 1,4-naphtoquinone (0,01 mole) et du 3-méthylindole (0,4 eq), un pigment noir.

EXEMPLE DE PREPARATION 16

Dans un tricol de 50 ml, muni d'un réfrigérant, d'un thermomètre et d'une agitation magnétique, on place 1,6 g (0,01 mole) de 1,4-naphtoquinone et 1,6 g de talc dans 16 ml d'acide acétique. On chauffe sous agitation à la température de 70°C puis on ajoute 0,52 g (0,4 eq) de N-méthylindole. Après 2 heures 30 minutes, on refroidit, on filtre et on lave le pigment dans des solvants de polarités différentes (eau, éthanol, acétone) jusqu'à l'obtention d'un filtrat non coloré.

On obtient un pigment brun.

EXEMPLE DE PREPARATION 17

Dans un tricol de 50 ml, muni d'un réfrigérant, d'un thermomètre et d'une agitation magnétique, on place 1,74 g (0,01 mole) de 5-hydroxy 1,4-naphtoquinone et 1,74 g de talc dans 17,4 ml d'acide acétique. On chauffe sous agitation à la température de 70°C puis on ajoute 0,78 g (0,4 eq) de 2-phénylindole. Après 24 heures, on refroidit, on filtre et on lave le pigment dans des solvants de polarités différentes (eau, éthanol, acétone) jusqu'à l'obtention d'un filtrat non coloré.

On obtient un pigment vert.

EXEMPLE DE PREPARATION 18

Selon le même protocole opératoire que l'exemple 17, on obtient, à partir de la 1,4-naphtoquinone et du 3-méthylindole (2 eq), un pigment vert.

EXEMPLE DE PREPARATION 19

Dans un tricol de 50 ml, muni d'un réfrigérant, d'un thermomètre et d'une agitation magnétique, on place 1,75 g (16,2 mmoles) de 1,4-benzoquinone et 1,75 g de talc dans 25 ml d'acide acétique. On agite, on chauffe à reflux puis on ajoute 0,6 g (3,2 eq) d'indole. Après 30 minutes, on refrodiit, on filtre et on lave le pigment dans des solvants de polarités différentes (eau, éthanol, acétone) jusqu'à l'obtention d'un filtrat non coloré.

On obtient un pigment grenat.

EXEMPLE DE PREPARATION 20

Dans un tricol de 100 ml, muni d'un réfrigérant, d'un thermomètre et d'une agitation magnétique, on place 1,5 g (13,9 mmoles) de 1,4-benzoquinone et 1,5 g de talc dans 45 ml d'acide acétique. On agite à la température ambiante puis on ajoute 2,25 g de 2-méthylindole (1,2 eq). Après 24 heures, on filtre et on lave le pigment dans des solvants de polarités différentes (eau, éthanol, acétone) jusqu'à l'obtention d'un filtrat non coloré.

On obtient un pigment bleu.

EXEMPLE DE PREPARATION 21

Dans un tricol de 50 ml, muni d'un réfrigérant, d'un thermomètre et d'une agitation magnétique, on place 1 g (3,8 mmoles) de 1,2-naphtoquinone 4-sulfonate de sodium et 1 g de talc dans un mélange acide acétique-eau (1-1). On chauffe sous agitation à la température de 70°C puis on ajoute 0,9 g (2 eq) d'indole. Après 24 heures, on refroidit, on filtre et on lave le pigment dans des solvants de polarités différentes (éthanol, acétone) jusqu'à l'obtention d'un filtrat non coloré.

On obtient un pigment ocre.

EXEMPLE DE PREPARATION 22 (COMPARATIF)

Dans un tricol de 50 ml, muni d'un réfrigérant, d'un thermomètre et d'une agitation magnétique, on dissout 1,6 g de 1,4-naphtoquinone dans 16 ml d'éthanol à la température de 70°C. Puis on ajoute 0,52 g (0,4 eq) de 2-méthylindole. Lorsque la réaction n'évolue plus, on ajoute 1,6 g de talc. Après 24 heures, on refroidit, on filtre et on lave le pigment dans des solvants de polarités différentes (éthanol, acétone) jusqu'à l'obtention d'un filtrat non coloré.

On obtient un pigment gris.

Ce pigment coloré obtenu par adsorption du colorant formé par couplage de l'indole et de la quinone, a une couleur tout à fait différente de celui obtenu à l'exemple 5.

EXEMPLE DE PREPARATION 23

Dans un tricol de 50 ml, muni d'un réfrigérant, d'un thermomètre et d'une agitation magnétique, on place 1,6 g (0,01 mole) de 1,4-naphtoquinone et 1,6 g d'alumine neutre (granulométrie 3,75 μm) dans 16 ml d'acide acétique. On chauffe sous agitation à la température de 70°C puis on ajoute 3,55 g (3 eq) d'indole. Après 48 heures, on refroidit, on filtre et on lave le pigment dans des solvants de polarités différentes (eau, éthanol, acétone et dichlorométhane) jusqu'à l'obtention d'un filtrat non coloré. On sèche à chaud et on obtient un pigment de couleur rouge.

EXEMPLE DE PREPARATION 24 (COMPARATIF)

Dans un tricol de 50 ml, muni d'un réfrigérant, d'un thermomètre et d'une agitation magnétique, on dissout 1,6 g (0,01 mole) de 1,4-naphtoquinone dans 16 ml d'acide acétique à la température de 70°C. On ajoute 3,55 g (3 eq) d'indole et après 24 heures de réaction à cette température, on introduit 1,6 g d'alumine neutre (granulométrie 3,75 μm). On agite à nouveau 24 heures. On refroidit, on filtre et on lave le pigment dans des solvants de polarités différentes (eau, éthanol, acétone et dichlorométhane) jusqu'à l'obtention d'un filtrat non coloré. On obtient après séchage, un pigment de couleur rouge.

Le tableau I ci-après illustre l'influence de l'adsorption (exemple 24) par rapport à la réaction in situ conforme à l'invention (exemple 23) sur la granulométrie du pigment final.

**TABLEAU I**

| Comparaison de la granulométrie (μ) des pigments rouges des exemples 23 et 24. | | |
|---|---|---|
| | Exemple 23 | Exemple 24 |
| Diamètre moyen (en volume) | 4,07 | 7,5 |
| Ecart type (en volume) | 3,26 | 10,18 |

Les valeurs d'écart-type des pigments obtenus dans ces 2 exemples, mettent en évidence la plus grande dispersion en taille du pigment obtenu dans l'exemple 24.

EXEMPLE DE PREPARATION 25 (COMPARATIF)

Dans un tricol de 100 ml, muni d'un réfrigérant, d'un thermomètre et d'une agitation magnétique, on dissout à la température de 70°C, 3,2 g (20 mmoles) de 1,4-naphtoquinone dans 32 ml d'acide acétique. Puis on ajoute 7,1 g d'indole (3 eq). Après 48 heures, on refroidit, on filtre et on lave le précipité rouge dans des solvants de polarités différentes (eau, éthanol, acétone) jusqu'à l'obtention d'un filtrat non coloré.

Cet exemple illustre l'obtention du colorant seul, non supporté sur charge inorganique.

Le tableau II ci-après résume les résultats obtenus.

| Exemples | SUBSTRAT | REACTIF | SUPPORT | MESURE DE LA COULEUR (L, a, b) |
|---|---|---|---|---|
| 1 | 1,4-naphto-quinone | Indole | Talc | L : 54,5<br>a : 27,0 Rouge<br>b : 13,0 |
| 2 | - | - | Talc | L : 54,3<br>a : 27,3 Rouge<br>b : 16,9 |
| 3 | - | - | Alumine neutre | Rouge |
| 4 | - | - | Alumine neutre | L : 61,9<br>a : 35,4 Rouge<br>b : 28,0 |
| 5 | - | 2-méthyl-indole | Talc | L : 46,9<br>a : 11,2 Violet<br>b : - 4,5 |
| 6 | - | | Alumine acide | L : 44,1<br>a : 17,7 Violet<br>b : - 2,3 |
| 7 | 5-hydroxy 2-méthyl 1,4-naphtoquinone | | Alumine acide | L : 39,5<br>a : 10,3 Violet<br>b : 5,1 |
| 8 | - | Indole | Alumine acide | L : 39,6<br>a : 12,9 Violet<br>b : 7,6 |
| 9 | 2-bromo 3-méthoxy 1,4-naphto-quinone | 2-méthyl-indole | Talc | L : 74,2<br>a : 8,5 Violet<br>b : - 7,2 |
| 10 | 2-hydroxy 1,4-naphto-quinone | - | Talc | L : 73,1<br>a : 10,8 Violet<br>b : - 0,8 |
| 11 | 5-hydroxy 1,4-naphto-quinone | Indole | Talc | L : 31,0<br>a : 0,0 Noir<br>b : 1,5 |
| 12 | - | - | Alumine acide | L : 31,5<br>a : 0,6 Noir<br>b : 1,8 |

| Exemples | Pigment N° | SUBSTRAT | REACTIF | SUPPORT | MESURE DE LA COULEUR (L, a, b) |
|---|---|---|---|---|---|
| 13 | | 5-hydroxy 1,4-naphto-quinone | 2-carboxy indole | Alumine acide | Noir |
| 14 | | 2,5-dihydroxy 1,4-naphto-quinone | 2-méthyl-indole | Alumine acide | Noir |
| 15 | | 5-hydroxy 1,4-naphto-quinone | 3-méthyl-indole | Talc | Noir |
| 16 | | 1,4-naphto-quinone | N-méthyl-indole | Talc | L : 40,2<br>a : 13,6<br>b : 6,6<br>Brun |
| 17 | | 5-hydroxy 1,4-naphto-quinone | 2-phényl-indole | - | L : 40,4<br>a : - 3,5<br>b : 8,4<br>Vert |
| 18 | | 1,4-naphto-quinone | 3-méthyl-indole | - | L : 43,3<br>a : - 0,8<br>b : 5,7<br>Vert |
| 19 | | 1,4-benzo-quinone | Indole | - | L : 37,1<br>a : 17,2<br>b : 7,7<br>Grenat |
| 20 | | - | 2-méthyl indole | - | L : 41,2<br>a : 10,3<br>b : - 5,6<br>Bleu |
| 21 | | 1,2-naphto-quinone 4-sulfonate de sodium | Indole | - | L : 67,7<br>a : 6,1<br>b : 17,0<br>Ocre |
| 22 | 2 | 1,4-naphto-quinone | 2-méthyl-indole | - | L : 60,9<br>a : 12,7<br>b : 7,0<br>Gris |
| 23 | 1 | - | Indole | Alumine neutre | L : 58,8<br>a : 27,4<br>b : 16,9<br>Rouge |
| 24 | 1 | - | - | Alumine neutre | L : 56,5<br>a : 29,7<br>b : 17,7<br>Rouge |
| 25 | | - | - | | L : 47,7<br>a : 35,6<br>b : 23,4<br>Rouge |

## EXEMPLES D'APPLICATION

### EXEMPLE D'APPLICATION 1

| | | |
|---|---|---|
| - Pigment de l'exemple 4 (obtenu par couplage de la 1,4-naphtoquinone avec l'indole sur alumine neutre) de granulométrie 3,75 µ | | 3 g |
| - Pigment de l'exemple 5 (couplage de la 1,4-naphtoquinone et du 2-méthylindole sur le talc) de granulométire 10 µ | | 6 g |
| - Béhénate d'octylglycéryl | | 11 g |
| - Triglycérides d'acides caprylique/caprique | | 11 g |
| - Cire microcristalline | | 15 g |
| - Lanoline liquide | | 17,50 g |
| - BHT | | 0,16 g |
| - Huile de ricin | qsp | 100 g |

L'ensemble des corps gras est fondu à 90-95°C. Les pigments sont incorporés à chaud, et la formule est broyée au tricylindre.

Le produit obtenu est une pâte souple, dans laquelle les pigments sont bien répartis (confirmé par l'observation au microscope).

Le produit est utilisé comme rouge à lèvres.

Le maquillage est de couleur bois de rose, brillant et transparent.

### EXEMPLE D'APPLICATION 2

| | | |
|---|---|---|
| - Pigment de l'exemple 11 (couplage de la 5-hydroxy 1,4-naphtoquinone avec l'indole sur du talc) de granulométrie 10 µ | | 7 g |
| - Octaméthylcyclosiloxane | | 25 g |
| - Glycérine | | 2 g |
| - Triéthanolamine | | 0,80 g |
| - Polymère carboxyvinylique modifié, vendu sous la dénomination de CARBOPOL EX 183 | | 0,10 g |
| - Polymère carboxyvinylique réticulé, vendu sous la dénomination de CARBOPOL 940 | | 0,6 g |
| - Conservateur | | 0,2 g |
| - Eau | qsq | 100 g |

Les polymères sont dispersés à chaud avec les conservateurs dans l'eau pour former un gel. La glycérine et la triéthanolamine sont alors ajoutées. Le pigment est dispersé dans la silicone et ajouté dans le gel. On obtient une émulsion gélifiée, brillante, noire, pour le maquillage des cils.

## Revendications

1. Produit sous forme de particules inorganiques colorées, caractérisé par le fait qu'il résulte de la réaction d'un dérivé indolique et d'un dérivé quinonique en présence desdites particules inorganiques dans un milieu approprié pour la réaction.

2. Produit selon la revendication 1, caractérisé par le fait que le dérivé indolique est choisi parmi les composés répondant à la formule :

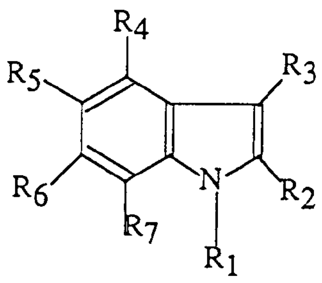

(I)

dans laquelle :

$R_1$ représente un atome d'hydogène ou un groupement alkyle inférieur en $C_1$-$C_4$;

$R_2$ et $R_3$, identiques ou différents, représentent un atome d'hydrogène, un groupement alkyle en $C_1$-$C_4$, phényle ou carboxyle;

$R_4$, $R_5$, $R_6$ et $R_7$, identiques ou différents, représentent un atome d'hydrogène, d'halogène, hydroxyle, un groupement alkyle ou alcoxy en $C_1$-$C_4$, alcoxy($C_1$-$C_4$)carbonyle, acyle en $C_2$-$C_4$, acyl($C_2$-$C_4$)oxy, cyano, amino éventuellement substitué par alkyle en $C_1$-$C_4$ ou hydroxyalkyle en $C_1$-$C_4$, $NO_2$, benzyloxy, $R_6$ et $R_6$ pouvant former un cycle méthylènedioxy, un seul au plus des radicaux $R_4$ à $R_7$ peut désigner halogène, $NO_2$, alcoxycarbonylé, acyle, cyano, amino, deux au plus des radicaux $R_4$ à $R_7$ peuvent désigner OH, alcoxy, benzyloxy ou acyloxy.

**3.** Produit selon la revendication 1 ou 2, caractérisé par le fait que le dérivé quinonique est choisi parmi les 1,2 et 1,4-benzoquinones et naphtoquinones, répondant aux formules :

a)

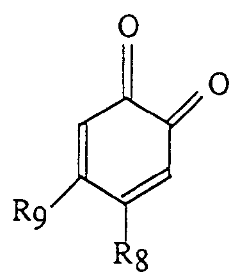

(II)

dans laquelle $R_8$ et $R_9$ désignent, indépendamment l'un de l'autre, un atome d'hydrogène ou d'halogène, un radical alkyle en $C_1$-$C_4$ ou alcoxy en $C_1$-$C_4$ ou le groupe $SO_3M$, M représentant un atome d'hydrogène, un métal alcalin, ammonium ou un reste d'amine;

b)

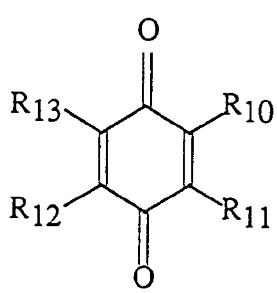

(III)

dans laquelle $R_{10}$ désigne un atome d'hydrogène, un atome d'halogène, un radical alkyle en $C_1$-$C_4$, alcoxy en $C_1$-$C_4$, hydroxyalkyle en $C_1$-$C_4$, acyl($C_1$-$C_4$)amino, alcoxy($C_1$-$C_4$)carbonyle, hydroxyalcoxy($C_1$-$C_4$) car-

boxyalkyle($C_1$-$C_4$), carboxyalcoxy($C_1$-$C_4$), alcoxy($C_1$-$C_4$) alkyle($C_1$-$C_4$), hydroxyalkyl($C_1$-$C_4$)thio, polyhydroxyalkyl($C_1$-$C_4$) thio, carboxyalkyl($C_1$-$C_4$)thio, alkyl($C_1$-$C_4$)thio, mono- ou dialkyl ($C_1$-$C_4$)amino, hydroxyalkyl($C_1$-$C_4$)thioalkyl($C_1$-$C_4$), alkyl($C_1$-$C_4$) thioalkyle($C_1$-$C_4$), $SO_3M$, M ayant la signification ci-dessus;

$R_{11}$, $R_{12}$, $R_{13}$ désignent, indépendamment l'un de l'autre, un atome d'hydrogène, d'halogène, un radical alkyle en $C_1$-$C_4$, alcoxy en $C_1$-$C_4$, hydroxyalkyl($C_1$-$C_4$)thio, acylamino,

et au moins l'un des radicaux $R_{10}$ à $R_{13}$ désigne hydrogène ;

c)

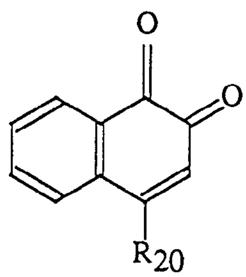

(IV)

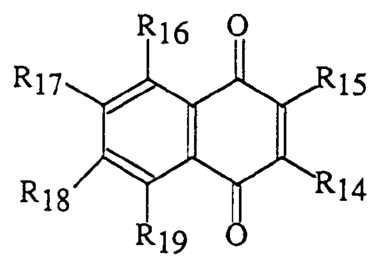

(V)

dans laquelle :

$R_{16}$ à $R_{19}$, identiques ou différents, désignent un atome d'hydrogène, un groupement OH, un groupement alcoxy en $C_1$-$C_4$, deux groupements au plus pouvant désigner OH;

$R_{14}$ et $R_{15}$ désignent un atome d'hydrogène, un groupement OH, un atome d'halogène, un radical alkyle en $C_1$-$C_{12}$, alcoxy en $C_1$-$C_4$, phényle, thiophényle, adamantylméthyle, adamantylamino, alkyl($C_1$-$C_4$)amino, pipéridyle, morpholinyle;

$R_{20}$ représente un atome d'hydrogène ou un groupe $SO_3M$, M ayant la signification ci-dessus.

4. Produit selon l'une quelconque des revendications 1 à 3, caractérisé par le fait que les particules inorganiques sont choisies parmi les particules solides à base d'oxyde de magnésium, d'oxyde de fer, d'oxyde de silicium, d'oxyde d'aluminium, d'oxyde de titane, d'oxyde de chrome, d'oxyde de zinc, d'oxyde de manganèse, d'oxychlorure de bismuth, de carbonate de magnésium.

5. Produit selon la revendication 4, caractérisé par le fait que les particules inorganiques sont choisies parmi le talc, les alumines, le kaolin, le carbonate de magnésium, les oxydes de titane, les oxydes de zinc, les oxydes de chrome, les oxydes de fer, le mica, la silice.

6. Produit selon l'une quelconque des revendications 1 à 5, caractérisé par le fait que les dérivés indoliques sont choisis parmi l'indole, le N-méthylindole, le 2-méthylindole, le 3-méthylindole, le 2-phénylindole, le 2-carboxyindole.

7. Produit selon l'une quelconque des revendications 1 à 6, caractérisé par le fait que les dérivés quinoniques sont choisis parmi la 1,4-benzoquinone, la 1,4-naphtoquinone, la 5-hydroxy 1,4-naphtoquinone, la 2-hydroxy 1,4-naphtoquinone, la 5-hydroxy 2-méthyl 1,4-naphtoquinone, la 3-méthoxy 2-bromo 1,4-naphtoquinone, la 2,5-dihydroxy 1,4-naphtoquinone, le 4-sulfonate 1,2-naphtoquinone de sodium.

8. Procédé de préparation d'un produit à base de particules inorganiques colorées, caractérisé par le fait que l'on fait réagir un dérivé quinonique et un dérivé indolique tels que définis dans l'une quelconque des revendications 1 à 7, en présence de particules inorganiques, dans un milieu approprié pour la réaction.

9. Procédé selon la revendication 8, caractérisé par le fait que le milieu est constitué d'un milieu éthanolique ou un mélange d'eau et d'acide acétique ou de l'acide acétique.

10. Utilisation du produit tel que défini dans l'une quelconque des revendications 1 à 7 ou préparé selon le

procédé défini dans l'une quelconque des revendications 8 et 9 comme pigment dans l'industrie alimentaire.

**11.** Utilisation du produit tel que défini dans l'une quelconque des revendications 1 à 7 ou préparé selon le procédé défini dans l'une quelconque des revendications 8 et 9 comme pigment dans des peintures.

**12.** Utilisation du produit tel que défini dans l'une quelconque des revendications 1 à 7 ou préparé selon le procédé défini dans l'une quelconque des revendications 8 et 9, en cosmétique.

**13.** Composition cosmétique, caractérisée par le fait qu'elle comprend au moins en partie un pigment constitué par un produit tel que défini dans l'une quelconque des revendications 1 à 7, dans un milieu cosmétiquement acceptable.

**14.** Composition selon la revendication 13, destinée à être utilisée pour le maquillage de la peau, des cils, des sourcils ou des ongles, pour la coloration temporaire des cheveux, caractérisée par le fait qu'elle se présente sous forme de lotion, de lotion épaissie, de gel, de crème, de pommade, de poudre, de stick, de dispersion, de dispersion liposomale.

**15.** Composition selon l'une quelconque des revendications 13 et 14, caractérisée par le fait qu'elle contient en outre des corps gras, des solvants organiques, des silicones, des épaississants, des adoucissants, des tensio-actifs, des filtres solaires, des agents anti-mousse, des agents hydratants, des parfums, des conservateurs, des agents antioxydants, des charges, des séquestrants, des agents de traitement, des propulseurs, des agents alcalinisants, acidifiants ou d'autres pigments.

**16.** Utilisation de la composition telle que définie dans l'une quelconque des revendications 13 à 15, pour le maquillage de la peau, la protection de l'épiderme humain contre les effets néfastes du rayonnement UV, pour la coloration des cheveux ou des ongles.

Office européen des brevets

# RAPPORT DE RECHERCHE EUROPEENNE

Numero de la demande

EP 93 40 0872

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | EP-A-0 460 996 (L'OREAL)<br>* page 3 *<br>* page 5 *<br>* abrégé *<br>--- | 1-3, 12-14 | A61K7/00<br>A61K7/13<br>C09C3/08<br>C09B67/08<br>C09B63/00 |
| A | EP-A-0 467 767 (L'OREAL)<br>* page 2, ligne 50 - page 4, ligne 10 *<br>* abrégé *<br>--- | 1,2,4-6, 12-16 | |
| D,A | FR-A-2 626 173 (L'OREAL)<br>* le document en entier *<br>--- | 1-3, 12-14 | |
| D,A | TETRAHEDRON LETTERS<br>no. 15, 4 Mars 1974, LONDON, GB<br>pages 1361 - 1362<br>PRASAD ET. AL. 'A Reinvestigation of the Reaction of Indole with 1,4-Quinons'<br>* page 1361 - page 1362 *<br>--- | 2,3 | **DOMAINES TECHNIQUES RECHERCHES (Int. Cl.5)** |
| A | GB-A-2 207 153 (L'OREAL)<br>* le document en entier *<br>----- | 1 | A61K<br>C09C<br>C09B |

Le présent rapport a été établi pour toutes les revendications

| Lieu de la recherche | Date d'achèvement de la recherche | Examinateur |
|---|---|---|
| LA HAYE | 27 JUILLET 1993 | KETTERER M. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)